# EUROPEAN PATENT SPECIFICATION

(11) **EP 3 344 653 B1**
(45) Date of publication and mention of the grant of the patent: **18.11.2020**
(21) Application number: 16778879.3
(22) Date of filing: 01.09.2016
(51) Int. Cl.: C07K 14/79

(54) **A COMPLEX OF LACTOFERRIN WITH MANGANESE IONS, A METHOD OF MANUFACTURING USE AND A PHARMACEUTICAL COMPOSITION COMPRISING COMPLEX OF LACTOFERRIN WITH MANGANESE IONS**
KOMPLEX AUS LACTOFERRIN MANGANIONEN, VERFAHREN ZUR HERSTELLUNG, VERWENDUNG UND PHARMAZEUTISCHE ZUSAMMENSETZUNG MIT DEM KOMPLEX AUS LACTOFERRIN MIT MANGANIONEN
COMPLEXE DE LACTOFERRINE AVEC DES IONS MANGANÈSE, PROCÉDÉ DE FABRICATION ET D'UTILISATION ET COMPOSITION PHARMACEUTIQUE COMPRENANT LE COMPLEXE DE LACTOFERRINE AVEC DES IONS MANGANÈSE

(30) Priority: 04.09.2015 PL 41383015
(43) Date of publication of application: 11.07.2018
(73) Proprietor: Uniwersytet Jagiellonski, 31-007 Krakow (PL)
(72) Inventor: STRUS, Magdalena, 30-617 Kraków (PL); STOCHEL, Grazyna, 31-235 Kraków (PL); BRINDELL, Malgorzata, 30-348 Kraków (PL); PILARCZYK-ZUREK, Magdalena, 30-347 Kraków (PL); SPIEWAK-WOJTYLA, Klaudyna, Wieliczka 32-020 (PL); MAJKA, Grzegorz, 30-334 Kraków (PL)
(74) Representative: BRANDPAT Patent and Trademark Attorneys Chlebicka Czyz Galazkiewicz Ziolkowski Professional Partnership
(86) International application number: PCT/IB2016/055218
(87) International publication number: WO 2017/037641

(56) References cited:
- EP-A1- 0 389 795
- GOLDONI PAOLA ET AL: "Metal complexes of lactoferrin and their effect on the intracellular multiplication of Legionella pneumophila", BIOMETALS, vol. 13, no. 1, March 2000 (2000-03), pages 15-22, XP002764120, ISSN: 0966-0844
- Radoslaw Rudz: "MANGANESE-LACTOFERRIN COMPLEX WITH PROPERTIES STIMULATING THE GROWTH OF PROBIOTIC BACTERIA", , 9 December 2015 (2015-12-09), XP055317855, Retrieved from the Internet: URL:http://www.innoget.com/uploads/32bf2c6 0ad6c1d117841b87c6f4966844db23ede.pdf [retrieved on 2016-11-09]
- TURIN CHRISTIE G ET AL: "Lactoferrin for prevention of neonatal sepsis", BIOMETALS, KLUWER ACADEMIC PUBLISHERS, NL, vol. 27, no. 5, 17 June 2014 (2014-06-17), pages 1007-1016, XP035385406, ISSN: 0966-0844, DOI: 10.1007/S10534-014-9754-3 [retrieved on 2014-06-17]
- PAOLO MANZONI ET AL: "Lactoferrin for prevention of neonatal infections", CURRENT OPINION ON INFECTIOUS DISEASES., vol. 24, no. 3, 1 June 2011 (2011-06-01), pages 177-182, XP055318158, GB ISSN: 0951-7375, DOI: 10.1097/QCO.0b013e32834592e6
- VALENTI P ET AL: "Lactoferrin; Lactoferrin: an important host defence against microbial and viral attack", CMLS CELLULAR AND MOLECULAR LIFE SCIENCES, BIRKHÄUSER-VERLAG, BA, vol. 62, no. 22, 1 November 2005 (2005-11-01), pages 2576-2587, XP019200890, ISSN: 1420-9071, DOI: 10.1007/S00018-005-5372-0
- Majka et al.,: "The impact of lactoferrin with different levels of metal saturation on the intestinal epithelial barrier function and mucosal inflammation | Springer for Research & Development", , 18 October 2016 (2016-10-18), XP055318673, Retrieved from the Internet: URL:http://rd.springer.com/article/10.1007 /s10534-016-9973-x [retrieved on 2016-11-11]

## Description

The subject-matter of the invention is a complex of lactoferrin with manganese (III) ions, procedures required for its preparations and potential uses - especially for production of dietary supplement and/or pharmaceutical composition for treatment and prevention of conditions associated with gut microbiota, as well as a pharmaceutical composition that contains lactoferrin-manganese(III) complex itself.

Development of medicine within the last few decades led to vast improvement of medical care provided both for pregnant women and the neonates. Hygienic status and formation of modern neonatology is what allowed doctors to protect the lives of the preterm neonates even with a very low-birth weight (<1500 g, VLBW).

Upon natural labor, neonate's body becomes colonized with natural bacterial microflora (mostly of *Lactobacillus* and *Bifidobacterium* genera) coming from the mother's urogenital and gastrointestinal tract. It has been proven that microbiota acquired in this way is one of the first defense mechanisms protecting the neonate from the pathogenic microorganisms present in a hospital environment. In the case of the preterm infants treated in the intensive neonatal care units, the situation is vastly different. Such neonates, separated from their mothers, are either given formulas or fed parenterally and are at particular risk of exposure and colonization with nosocomial bacterial strains having high virulence potential. Moreover, VLBW infants not fed with mother's milk not only do not receive the natural microbiota (*Bifidobacterium* and *Lactobacillus* species) but also other nutritional components, growth factors, cytokines, natural antibacterial peptides, soluble IgA antibodies etc. Bacteria from the hospital environment may divide without any control in the intestinal lumen leading to gut dysbiosis. Improperly developed intestinal mucous barrier having low motility and dysfunctional immune system give a way to bacterial toxins and antigens abundance. Loss of intestinal integrity leads to the translocation of the bacteria from the intestinal lumen to bloodstream resulting in systemic inflammatory response [Sherman MP. New Concepts of Microbial Translocation in the Neonatal Intestine: Mechanisms and Prevention. Clinics in Perinatology. 2010;37(3):565-79.]

Neonatal infections remain a major problem in the neonatal care contributing to infant morbidity and mortality [Lawn JE, Wilczynska-Ketende K, Cousens SN. Estimating the causes of 4 million neonatal deaths in the year 2000. Int J Epidemiol. 2006;35(3):706-18].

Feeding of neonates seems particularly significant in the context of prevention of bacterial translocation and subsequent bacteriemia. Importance of the naturally ocurring milk proteins can be observed upon comparison of the gut microbiota between infants fed with the mother's milk and infant formulae. Special regard is attributed to lactoferrin milk protein with GRAS (generally regarded as safe) status - its implicated beneficial properties made it an attractive issue investigated by many research groups.

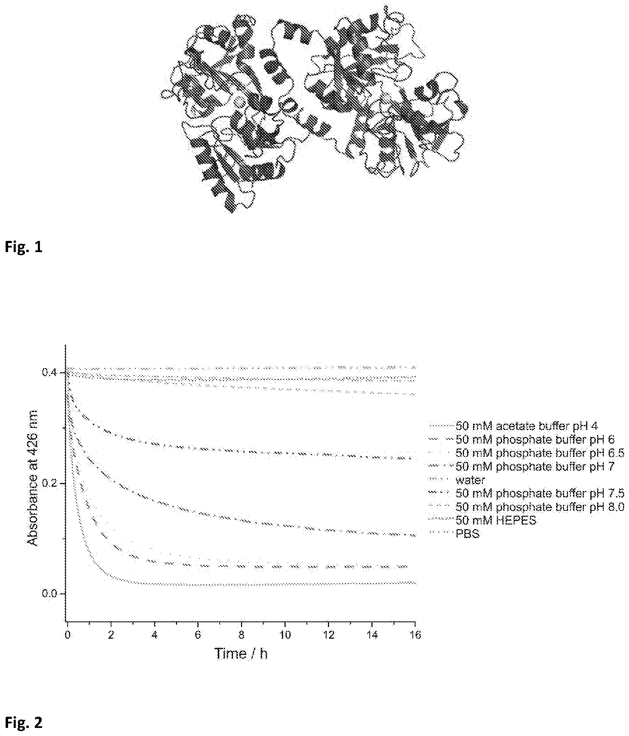

Lactoferrin (Lf) is a protein belonging to the transferrin family, present in great abundance in mammalian secretions such as milk, saliva, tears, semen etc. Lactoferrin is a glycoprotein with a molecular weight of ca. 80 kDa. Its structure comprises of a single polypeptide chain folded into 2 homologous lobes (N- and C-lobe, Fig. 1).

Lactoferrin has growing attention due to its antibacterial, antifungal and antiviral properties that made it a suitable component for both dietary supplements and medical products [Sanchez L, Calvo M, Brock JH. Biological role of lactoferrin. Archives of Disease in Childhood. 1992;67(5):657-61]. Lactoferrin is in the center of interest when it comes to prevention of neonates, infants and older children [Huang J, Nandi S, Wu L, Yalda D, Bartley G, Rodriguez R, et al. Expression of natural antimicrobial human lysozyme in rice grains. Molecular Breeding. 2002;10(1-2):83-94]. Oral supplementation with various lactoferrin forms has considerable importance in mitigating of the symptoms of bacterial translocation from the intestinal lumen into the bloodstream that is a major cause of the neonatal sepsis in the VLBW infants.

Document US 2011/0183008 discloses a method for osteoporosis treatment that includes supplementation with lactoferrin comprising no more than 2 metal ions per lactoferrin molecule. Metals used included manganese, iron, copper, chromium, cobalt, zinc and magnesium.

Document WO 2011/50395 discloses a preparation containing lactoferrin and a nutritional mixture, used as a dietary supplement for pregnant women, women trying to get pregnant or women post-parturition. One or more ions per lactoferrin molecule are described and metals usedare: iron(III), zinc, copper and manganese.

Document EP2653040 discloses a nutritional composition for people with allergy to milk, especially preterm neonates and infants as a way to mitigate the influence of pathogenic microorganisms. Composition is based on donkey milk, raw or lyophilised with the addition of a lipid mixture. The composition contains lactoferrin and manganese.

Document WO 2011/50393 discloses a method for prevention or treatment of infantile colic involving infant supplementation with either mother's milk with isolated lactoferrin or combination of mother's milk and composition comprising lactoferin. Lactoferrin used is saturated with one or more iron(III), zinc, copper or manganese ions.

There have been reports on lactoferrin being able to bind manganese following removal of ferric ions [Marchetti M, Superti F, Ammendolia MG, Rossi P, Valenti P, Seganti L. Inhibition of poliovirus type 1 infection by iron-, manganese and zinc saturated lactoferrin. Medical Microbiology and Immunology. 1999;187(4):199-204, Ainscough EW, Brodie AM, Plowman JE. The chromium, manganese, cobalt, and copper complexes of human lactoferrin. Inorganica Chimica Acta. 1979;37(C):282].

The aim of the present invention is to demonstrate a lactoferrin complex resolving a technical problem by promoting growth of the probiotic bacteria while retaining the ability to bind iron the essential metal for growth of the pathogenic bacteria. Complex of lactoferrin and manganese ions (especially manganese(III)) do not have harmful impact on human cells and do stimulate the growth of tested probiotic strains. Increasing population number of the probiotic bacteria might indirectly contribute to suppresion of pathogenic microorganisms growth and their subsequent translocation from the intestinal lumen to the bloodstream.

The invention implements the aim by providing a complex of lactoferrin with mangese ions (LfMn) that do not contain ferric ions as well as a method for obtaining of such complex. This complex properties might be used for prevention of sepsis in the neonates. The invention might also serve as a prebiotic as a compononent of symbiotic preparations. Such preparation might also be used in treatment of chronic inflammatory bowel diseases (IBD). The invention could also be used as a dietary supplement for prevention and treatment of infections (of bacterial and viral origin). Lactoferrin preparations used hitherto have unspecified saturation with ferric ions.

An advantage of the invention consists in the presence of solely manganese in the complex with lactoferrin which also implicates its use in manganese supplementation. Manganese is an important microelement, a cofactor in many enzymes such as superoxide dysmutase that protects cells from the oxidative stress. Clinical symptoms of manganese deficits include suppression of mucopolysaccharides (like hyaluronian acid) and lipopolysaccharides synthesis, impairment of skeletal system development and ataxia. However, hypermanganism is dangerous as well leading to catecholamine exhaustion in the central nervous system, insomnia, headaches, anxiety, rapid hand movements, loss of movement coordination resembling Parkinson's disease. Manganese supplementation is advised in the case of the preterm newborns - mangangese is administered parenterally which might lead to complications associated with brain and liver disfunction [Rao R, Georgieff M. Nutrition of the Preterm Infant: Scientific Basis and Practical Guidelines. 0000:277-310 i Santos D, Batoreu C, Mateus L, Marreilha dos Santos AP, Aschner M. Manganese in human parenteral nutrition: Considerations for toxicity and biomonitoring. NeuroToxicology. 2014;43:36-45]. Maternal milk delivers 3 -10 µg/L during early infancy of which only 8% gets absorbed by the organism [J. Aschner. Nutritional aspects of manganese homeostasis. Molecular Aspects of Medicine 26, 2005]. However, despite excess of manganese in the mother's milk, the metal does not have adverse effects. Thus, the natural form of delivery allows for absorption of only the required amount of manganese. The excess of Mn ions undergoes gastrointestinal passage and might be utilised by probiotic bacteria and therefore facilitate formation of the proper intestinal microbiota. Manganese in a chelated form within complex with lactoferrin might be administered in an enteric coating and become absorbed in the gut where the manganese absorption normally occurs. The advantage of such approach is gradual release of manganese due to hight stability of MnLf complex in the basic pH of the gut. Administering of the complex in a form protecting form sudden local increase of manganese ions conecentration would contribute to increasing safety and beneficial impact of therapy using MnLf complex.

Another aim of the invention is providing a method for obtaining the MnLf complex. The protocol for production of MnLf complex relies on the dialysis process.

The advantage of the method is the possibility of regulation of manganese saturation level by changing pH value in the dialysis buffer. Additionally, since the obtained complexes are stable in water it is possible to lyophilise them in a form not contaminated with buffer salts.

Utilising dialysis allows for continuous process of MnLf production with minimal iron contamination, presence of which lowers the efficiency of manganese saturation reaction.

The advantage of the invention is that the method described leads to obtaining of lactoferrin complex with manganese(III). Previous reports described fromation of lactoferrin - manganese complexes with manganese(II) using pH^{∼}9 but there are no results confirming this fact. Our results proven that at a pH higher than ^{∼}8.5 lactoferrin binds non-specifically to manganese(II) (on top of the specific binding with manganese(III)).

The invention disclosed in this document is an answer to demand for preparations that could prevent bacterial infections and benefit the proper natural gastrointestinal microbiota. In the era of emerging multidrug resistance of numerous pathogenic bacterial strains it is crucial to implement effective defense relying on stimulating the growth of the probiotic bacteria of Lactobacillus genus. Such preparations might be of particularly great use in treatment of VLBW infants that are at great risk of developing infections. However, it might also be beneficial for patients after antibiotic therapy, for oncologic patients as well as for people suffering from inflammatory bowel diseases.

The subject-matter of this invention is presented in the **Fig. 1** depicting structure of bovine Lf with 2 ferric ions bound in the protein metal-binding sites (PDB: 1BLF); **Fig. 2** shows MnLf complex stability over time in several solutions: water, phosphate-buffered saline (PBS) and buffer solutions: HEPES (pH 7.0), acetate buffer (pH 4), phosphate buffer (pH 6.0, 6.5, 7.0, 7.5 and 8.0); **Fig. 3** shows electrophoregrams obtained upon eletrophoresis of post-reaction mixture - reaction at pH 7.5 (-) and after dialysis of the reaction mixture to MilliQ water for 24 hours (-); **Fig. 4** depicts diagram obtained after electrophoretic separation of reaction products of apoLf with manganese at pH 7.5 before and after dialysis to 0.1 M NaHCO₃ and to water in the case of normal conditions; **Fig. 5** shows SDS-PAGE separation for MnLf, original (native) lactoferrin preparation (DMV), holo- and apolactoferrin; **Fig. 6** shows EPR spectra of MnLf obtained using pH 8.5 (-); pH 7.5 (-); apoLf with Mn²⁺ at a concentration allowing for 50% saturation of the protein, mixed right before EPR measurement (-); apoLf with Mn²⁺ at a concentration allowing for 5% saturation of the protein, mixed right before the EPR measurement (-); all samples are prepared in MilliQ water; **Fig. 7** shows release of manganese from MnLf and iron from holo-Lf in an experimental setting resembling passage through gastrointestinal tract (100% corresponds to initial saturation of MnLf); **Fig. 8** shows manganese release in a setting resembling gastrointestinal tract passage - analysis of lactoferrin ability to bind iron after manganese is released from MnLf complex - arrow indicates the moment when ferric ions are added to the experimental system; **Fig. 9** shows growth curves of standard *Lactobacillus plantarum* strain (left panel) and *L. plantarum* strain originating from a commercial probiotic preparation incubated with: apolactoferrin (A), native Lf (N), hololactoferrin (H) and lactoferrin saturated with manganese ions (Mn) at concentrations of 0.6, 5.0 and 40.0 mg/ml. Values on graphs correspond to the mean optical density (wavelength 620 nm) in time for triplicate samples; **Fig. 10** shows growth curves of standard *Lactobacillus rhamnosus* strain (left panel) and *L. rhamnosus* strain originating from a commercial probiotic preparation incubated with: apolactoferrin (A), native Lf (N), hololactoferrin (H) and lactoferrin saturated with manganese ions (Mn) at concentrations of 0.6, 5.0 and 40.0 mg/ml. Values on graphs correspond to the mean optical density (wavelength 620 nm) in time for triplicate samples; **Fig. 11** shows the immunofluorescent staining of tight-junction protein (occludin) in Caco-2 cells: control cells - a, apolactoferrin - b, native Lf - c, hololactoferrin - d, MnLf - e; **Fig. 12** shows level of pro-inflammatory cytokine release from murine monocyte/macrophage cells (J774A.1 cell line) cultured in 0,5% FBS medium and treated with 100 ng/ml LPS and/or various Lf forms (at a concentration of 5 mg/ml, A - apolactoferrin, N - native Lf, H - hololactoferrin, Mn - manganese-saturated lactoferrin). After 23 hours of incubation, supernatants are collected and IL-6 (top panel) and TNF-α (bottom panel) are assayed using ELISA.

The subject- matter of the invention is a complex of lactoferrin with manganese (III) ions characterized by presence of 96-98% molar ratio of manganese ions to all metal ions in the complex. Preferably, manganese (III) ions comprise 98% (molar) of all metal ions in the complex. Preferably, manganese (III) saturation of lactoferrin is 50 % or below. For best results, lactoferrin-manganese complex should contain Mn³⁺ ions with saturation of ca. 50%.

Another subject-matter of the invention is a method for obtaining MnLf complex involving reaction of Lf with manganese salt and purification of the complex characterized in that the reaction is carried out via dialysis.

Preferably the lactoferrin-manganese reaction is conducted via dialysis at pH within a range 7.0 - 7.5 with buffer solution chosen from: 20 - 100 mM HEPES, 100 mM NaCl, 25 mM NaHCO₃, at a temperature in the range of 30 - 40°C, manganese salt used: manganese(II) citrate, manganese : Lf molar ratio in the range of 5 to 20, reaction time 12 - 48 hours, purification of the complex via dialysis to ultrapure water for 10 - 24 hours at room temperature with 3 changes of water. Preferably the molar ratio of Mn²⁺ to citric acid is 3:2 in a buffer solution (pH 7.4). It is required that lactoferrin reaction with manganese salt is conducted in aerobic conditions. Preferably the buffer solution is 50 mM HEPES. Preferably reaction of Lf with manganese salt is conducted at temperature of 37°C. Preferably lactoferrin reaction with manganese salt is conducted with at least 5-fold molar excess of Mn ions to lactoferrin. Preferably the lactoferrin reaction with manganese salt leads to obtaining the protein bound solely to Mn(III).

Another subject-matter of the invention is potential use of MnLf complex described above for production of dietary supplement, pharmaceutical preparation for treatment and/or prophylaxis of diseases and conditions associated with gastrointestinal microbiota. It is particularly beneficial for cases of dysbacteriosis associated with antibiotic therapy, oncotherapy, preterm birth, inflammatory bowel diseases. It is particularly beneficial when administered to preterm infants.

It is particularly beneficial when the dietary supplement and/or pharmaceutical composition is administered as a means of regulation of the gastrointestinal microbiota to tip the balance towards the probiotic bacteria. It is particularly beneficial for the diseases/conditions caused by bacteria, fungi and/or viruses. It is particularly beneficial for sepsis prevention and treatment. It is also beneficial for patients treated with antibiotics. It is beneficial for oncological patients. It is beneficial as a dietary supplement for patients suffering from chronic inflammatory bowel diseases.

Another subject-matter of the invention is a pharmaceutical composition comprising the complex of lactoferrin and manganese ions described above, as well as a dietary supplement comprising the claimed complex.

### Examples

### Methods for obtaining of lactoferrin-manganese(III) complex:

### Example 1.

Lactoferrin-manganese(III) complex obtained by:
- preparation of apolactoferrin with 1-2% iron saturation, without any Mn ions, according to the procedure described in Majka G., Śpiewak K., Kurpiewska K., Heczko P., Stochel G., Strus M., Brindell M., Analytical and Bioanalytical Chemistry, 2013, 405, 15; 5191-5200
- apolactoferrin is dissolved in 20 ml of MilliQ water at concentration of 50 mg/ml. The solution is pipetted into a dialysis tubing with 10kDa cut-off value
- 50 mM HEPES, **pH 7.4** buffer is prepared with addition of 100 mM NaCl and 25 mM NaHCO₃
- 0,43 mM of manganese(II) citrate is dissolved in 2 liters of the prepared HEPES buffer (this corresponds to Lf:Mn²⁺ ratio of 1:20)
- dialysis of apolactoferrin to a buffer comprising manganese(II) citrate is conducted for 24 hours at temperature of 37°C
- in order to remove non-specifically bound manganese ions dialysis to MilliQ water is conducted for another 24 hours with 3 changes of water
- MnLf preparation is lyophilized and stored at 4°C

Result: manganese saturation - ca. 50%, only Mn³⁺ present, MnLf complex stable at physiological pH.

### Example 2.

Lactoferrin-manganese complex obtained by:
- preparation of apolactoferrin with 1-2% iron saturation, without any Mn ions, according to the procedure described in Majka G., Śpiewak K., Kurpiewska K., Heczko P., Stochel G., Strus M., Brindell M., Analytical and Bioanalytical Chemistry, 2013, 405, 15; 5191-5200
- apolactoferrin is dissolved in 20 ml of MilliQ water at concentration of 50 mg/ml. The solution is pipetted into a dialysis tubing with 10kDa cut-off value
- 50 mM HEPES, **pH 8.5** buffer is prepared with addition of 100 mM NaCl and 25 mM NaHCO₃
- 0,43 mM of manganese(II) citrate is dissolved in 2 liters of the prepared HEPES buffer (this corresponds to Lf:Mn²⁺ ratio of 1:20)
- dialysis of apolactoferrin to a buffer comprising manganese(II) citrate is conducted for 24 hours at temperature of 37°C
- in order to remove non-specifically bound manganese ions dialysis to MilliQ water is conducted for another 24 hours with 3 changes of water
- MnLf preparation is lyophilized and stored at 4°C

Result: manganese saturation - ca. 100%, Mn²⁺ and Mn³⁺ present, MnLf complex stable at physiological pH.

### Example 3.

Lactoferrin-manganese complex obtained by:
- preparation of apolactoferrin with 1-2% iron saturation, without any Mn ions, according to the procedure described in Majka G., Śpiewak K., Kurpiewska K., Heczko P., Stochel G., Strus M., Brindell M., Analytical and Bioanalytical Chemistry, 2013, 405, 15; 5191-5200
- apolactoferrin is dissolved in 20 ml of MilliQ water at a concentration of 50 mg/ml. The solution is pipetted into a dialysis tubing with 10kDa cut-off value
- 50 mM sodium acetate buffer, **pH 4.0,** is prepared with addition of 100 mM NaCl and 25 mM NaHCO₃
- 0,43 mM of manganese(II) citrate is dissolved in 2 liters of the prepared HEPES buffer (this corresponds to Lf:Mn²⁺ ratio of 1:20)
- dialysis of apolactoferrin to a buffer comprising manganese(II) citrate is conducted for 24 hours at temperature of 37°C
- in order to remove non-specifically bound manganese ions dialysis to MilliQ water is conducted for another 24 hours with 3 changes of water
- MnLf preparation is lyophilized and stored at 4°C.

Result: manganese saturation - 0%

### Example 4.

Lactoferrin-manganese complex obtained by:
- preparation of apolactoferrin with 1-2% iron saturation, without any Mn ions, according to the procedure described in Majka G., Śpiewak K., Kurpiewska K., Heczko P., Stochel G., Strus M., Brindell M., Analytical and Bioanalytical Chemistry, 2013, 405, 15; 5191-5200
- apolactoferrin is dissolved in 20 ml of MilliQ water at a concentration of 50 mg/ml. The solution is pipetted into a dialysis tubing with 10kDa cut-off value
- 50 mM HEPES, **pH 7.4** buffer is prepared with addition of 100 mM NaCl and 25 mM NaHCO₃
- 0,43 mM of manganese(II) citrate is dissolved in 2 liters of the prepared HEPES buffer (this corresponds to Lf:Mn²⁺ ratio of 1:20)
- dialysis of apolactoferrin to a buffer comprising manganese(II) citrate is conducted for **4 hours** at temperature of 37°C
- in order to remove non-specifically bound manganese ions dialysis to MilliQ water is conducted for another 24 hours with 3 changes of water
- MnLf preparation is lyophilized and stored at 4°C

Result: manganese saturation - ca. 30%, only Mn³⁺ present, MnLf complex stable in physiological pH.

### Stability of the complexes according to the invention

To assess the stability of the formed complex between lactoferrin and manganese(III), freshly prepared MnLf is dissolved in MilliQ water and various buffers. Such solutions are incubated for 16 hours at 37°C with absorbance at 426 nm monitored continuously. Fig. 2 Shows stability of the manganese(III) binding for MnLf dissolved in: water, phosphate-buffered saline (PBS), HEPES (pH 7.0), acetate (pH 4.0) and phosphate (pH 6.0, 6.5, 7.0, 7.5 and 8.0) buffers.

Clear correlation between manganese release and buffer composition. MnLf is found to be stable in neutral and acidic solutions which do not contain ions that might form slightly soluble salts with manganese ions. Upon 16 hour incubation no manganese release is observed for MnLf dissolved in: MilliQ water, HEPES buffer (pH 7.0), PBS and phosphate buffer (pH 8.0). Increasing the acidity (lowering pH) of the phosphate buffer probably affect the protein conformation by increasing protonation of some amino acid residues which leads to weaker manganese binding. Increasing concentration of H⁺ correlates with levels of manganese released from Mn - the most efficient release is observed in acetate buffer, pH 4.0.

### Assessment of manganese saturation of lactoferrin

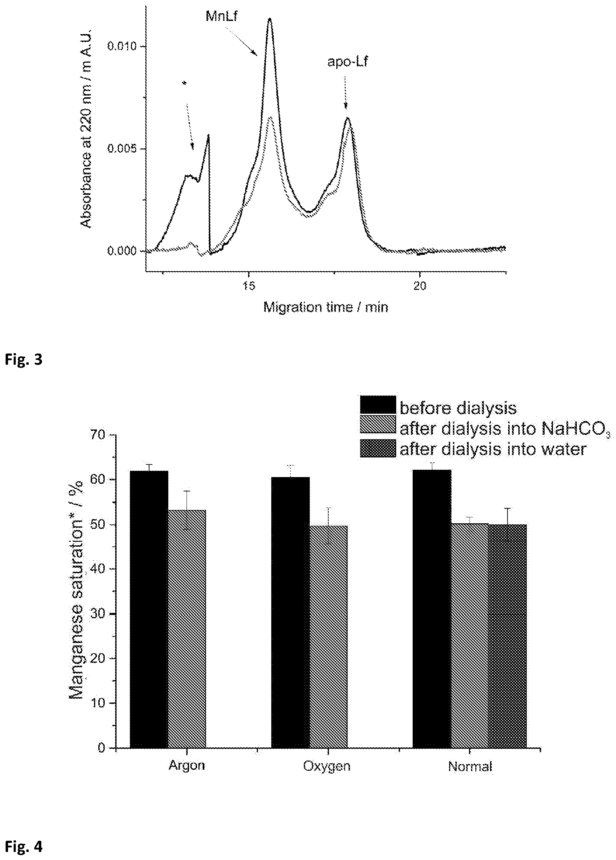

Quantitative determination of manganese and iron content in MnLf preparations is performed using ICP-OES (inductively-coupled plasma optical emission spectroscopy) with previously mineralized samples (mineralization using 65% ultrapure nitric acid). Lactoferrin concentration is determined using ELISA. **Table 1** summarizes the results of ICP-OES/ELISA measurements for three independently obtained MnLf samples. Lactoferrin : manganese molar ratio is close to 1 which corresponds to 50% saturation of metal-binding sites of the protein. Such results is in concord with data obtained upon capillary electrophoresis separation of MnLf samples **(****Fig. 3** and **Fig. 4****).**

**Table 1. Manganese content in 3 independent MnLf samples**

| **Sample** | **pH** | **Mn/Lf [M/M]** | **Mean saturation [%]** |
|---|---|---|---|
| **1** | 7.5 | 0,99 | 47±2 |
| **2** | | 0,92 | |
| **3** | | 0,91 | |
| **1** | 8.5 | 2,55 | 127±1 |
| **2** | | 2,56 | |
| **3** | | 2,53 | |

### Purity analysis of the complex according to the invention

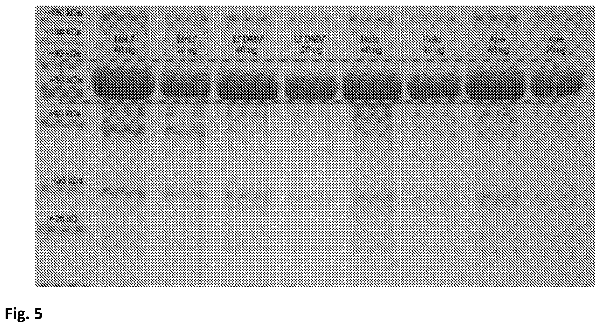

Lactoferrin abundance in the preparation is evaluated using SDS-PAGE. Fig. 5 depicts SDS-PAGE separation of: MnLf, original (native) lactoferrin (DMV), holo- and apolactoferrin. This allows for comparison of the preparations purity. We assessed the MnLf lactoferrin to be 90% pure based upon the electrophoretic separation. Therefore, the method used for obtaining manganese-saturated lactoferrin did not cause protein degradation.

### Assessment of manganese oxidation state in the prepared MnLf complexes

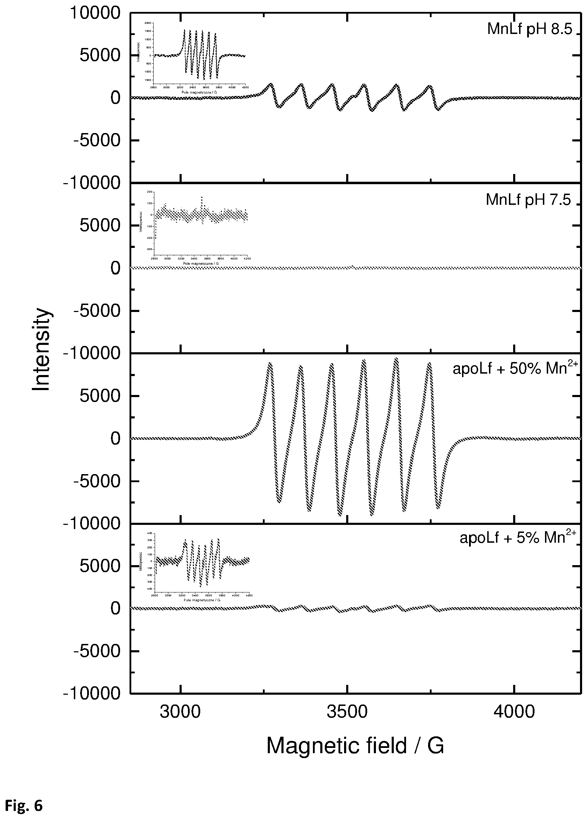

EPR spectra allowed for evaluation of the oxidation state of manganese in MnLf preparations. Conducted measurements have proven that only Mn³⁺ is present for MnLf prepared in using pH 7.5 buffer, we observed very little peak corresponding to Mn²⁺ in the EPR spectra (Fig. 6). On the contrary, the signal from Mn²⁺ is much more pronounced in the MnLf prepared using pH 8.5 buffer. Signal intensity analysis resulted in estimation that ca. 20% of manganese in the preparation is Mn(II). Manganese(III) is not visible in the EPR spectra but we may assume it is present in the sample due to relatively low intensity of Mn²⁺ signal in contrast to ICP-OES/ELISA (Table 1). Obtained results confirm that unspecific binding of manganese occurs at pH 8.5 and higher. This is also confirmed by higher than 100% manganese saturation of MnLf obtained in such conditions (Table 1). Furthermore, previously reported methods described reaction in 0.1 M carbonate buffer, pH of which is ca. 9 at room temperature. In such conditions, one can expect even higher levels of unspecific Mn²⁺ binding to the lactoferrin.

### Manganese release in the model of gastrointestinal tract passage

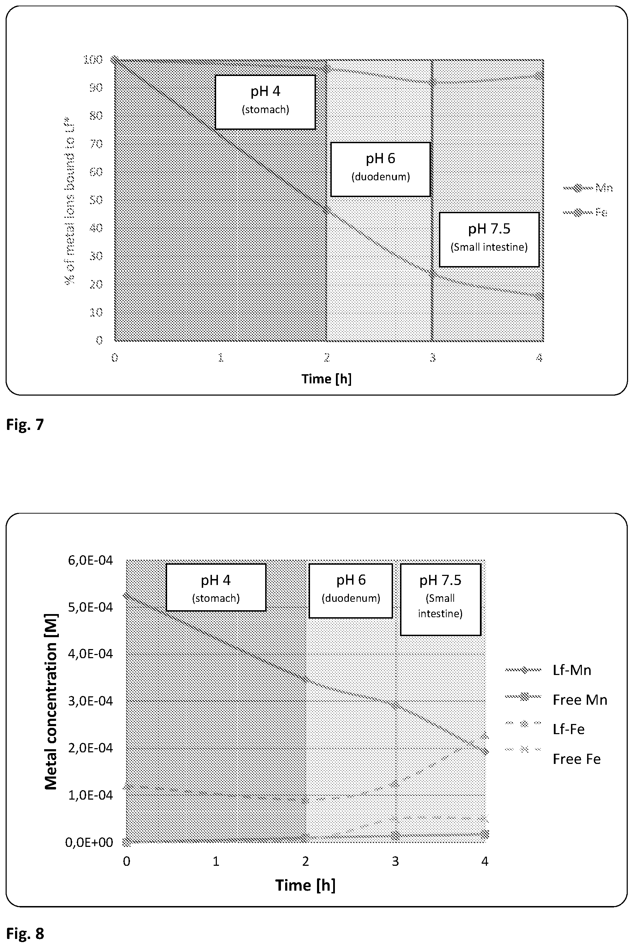

Determination of how manganese is released in conditions resembling the preterm infant gastrointestinal tract passage is performed using dialysis of 10 ml of 50 mg/ml MnLf to 200 ml of solution of hydrochloric acid at pH 4.0 for 2 hours. After addition of NaOH to the solution to reach pH 6.0, lactoferrin in the dialysis tubing is incubated for another hour. Following that incubation, pH is further increased to 7.5 and another incubation step is carried out for 1 hour. 100 µl of protein sample is collected on every step and mineralized using 65% ultrapure nitric acid. Mn and Fe content is evaluated using ICP-OES. The experiment is run simultaneously using holoLf for comparison. This setting in a simplified way corresponds to the passage through the gastrointestinal tract of the preterm infant. Gastric juice pH is not as low for preterm infants as for the term ones. Immediately after birth pH of the gastric juice is basic (within a range 7 - 8) but becomes acidic (2 - 3 range) within a few hours [C. Bourlieu. Specificity of Infant Digestive Conditions: Some Clues for Developing Relevant In Vitro Models. Critical Reviews in Food Science and Nutrition, 54:1427-1457 (2014)]. In the experiment described, it is assumed that MnLf might be administered within an hour after birth - resulting in an intermediate pH value of 4. Based on the literature data, time of residence in the stomach is set for 2 hours [C. Bourlieu. Specificity of Infant Digestive Conditions: Some Clues for Developing Relevant In Vitro Models. Critical Reviews in Food Science and Nutrition, 54:1427-1457 (2014)] (artificial formulae have 60 minutes half-life time in the infant stomach). Passage through the intestines is set for 2 hours [S. Bode. Gastric Emptying and Small Intestinal Transit Time in Preterm Infants: a Scintigraphic Method. Journal of Pediatric Gastroenterology and Nutrition 2004]. First hour correspond to the passage through the small intestine (pH 6.0) and second hour - large intestine, pH 7.5. Obtained results points to 50% manganese release within 2 hours of residence of MnLf in the acidic pH of the stomach. The remaining manganese-saturated Lf as well as the content of the stomach with the release manganese can be further transported to the small intestine where manganese is absorbed. Another experiment (Fig. 8) has proven that lactoferrin retains its iron-binding capability upon manganese release. As described in the previous experiment, similar incubation in the dialysis tubing is performed for MnLf. Dialysis is performed for 2 hours at pH 4.0 with following change of pH to 6.0 (using NaOH) and addition of ferric salt to the dialysis buffer in a molar ration allowing for 50% saturation of the protein placed in the dialysis tubing.

### Impact of various lactoferrin forms on growth of the probiotic strains of Lactobacillus genus

Performed experiments have assayed the impact of various lactoferrin forms (apolactoferrin, native Lf, hololactoferrin and MnLf) on 4 *Lactobacillus* strains. Testes strains are both standard strains: *Lactobacillus plantarum* (ATCC 14431) and *Lactobacillus rhamnsosus* GG (ATCC 53103) as well as strains of *L. plantarum* and *L. rhamnsosus* isolated from commercially available probiotic preparation (Lactoral, Biomed, Kraków, Poland).

To determine how various lactoferrin forms affect the growth of *Lactobacillus* strains the following procedure is employed: overnight culture of bacteria in 10 ml of MRS broth is centrifuged (10 minutes, 10 000 x g) and the bacterial pellet is resuspended in 10 ml of PBS. Rinsing with PBS is performed 2 times and the bacterial suspensions are then diluted to obtain ca. 10⁴ colony forming units per ml (cfu/ml) in the following media:
- MRS - positive control of *Lactobacillus* growth
- MRS-/- - MRS broth not comprising manganese and ferric ions. Metal ions are removed by 6 hours incubation of MRS broth with Chelex-100 resin which is then removed by filtration. MRS-/- served as a negative control for *Lactobacillus* growth
- MRS-/- with addition of various lactoferrin forms: A - apolactoferrin, N - native Lf, H - hololactoferrin, Mn - manganese-saturated lactoferrin at concentrations of 0.6, 5.0, 40.0 mg/ml

These bacterial suspensions are transferred (300 µl) to 96-well testing plates. Optical density at 620 nm is monitored during the culture (at temperature of 37°C) using Tecan M200 PRO microplate reader.

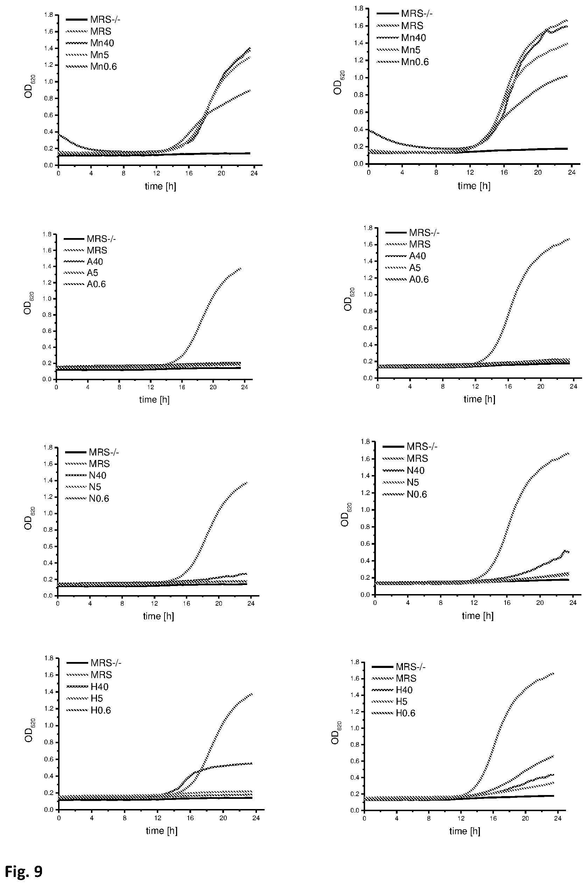

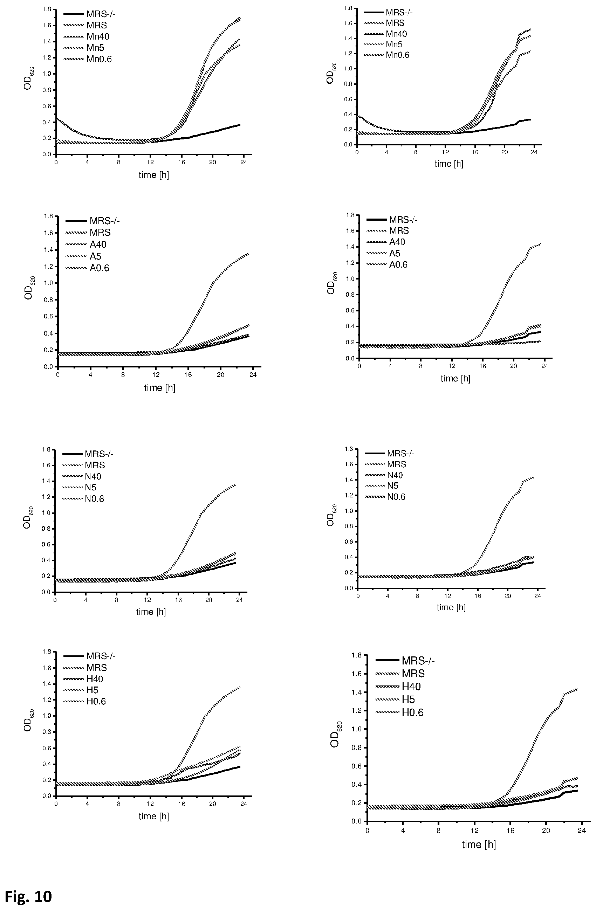

Obtained results (Fig. 9 and 10) unequivocally prove that only MnLf form promotes growth of all tested *Lactobacillus* strains. The growth kinetics observed in MnLf samples is similar to those registered for full MRS broth that also contain manganese ions. This is in concord with literature data on these bacteria - their growth is dependent upon manganese availability since manganese is present in their protein active sites in the place of iron. As opposed to MnLf, using other lactoferrin forms (apo-Lf, native Lf, holo-Lf) do not accelerate growth of the probiotic bacteria. This pose a major circumstance to recognize manganese-saturated lactoferrin as the only form of lactoferrin with beneficial impact on the bacteria of Lactobacillus genus. This conclusion presents a great advantage of the invention over commercially available and described in the literature lactoferrin preparations.

### MnLf complex impact on human intestinal epithelium cells

Caco-2 cell line is used to study the effect of manganese-saturated lactoferrin on viability and function on the intestinal epithelium barrier. Staining of the Caco-2 monolayer with annexin V-FITC and propidium iodide (Annexin-V-FLUOS Staining Kit, Roche) is performed to determine level of apoptotic and necrotic cells. No statistically significant difference are observed for cells treated with test lactoferrin forms at a concentration of 5 mg/ml (necrosis levels estimated to 1-2%, apoptosis - 0,5%). Lactoferrin saturated with manganese ions is not different in this aspect from the commercially available preparation. Immunofluorescent staining for tight junction protein (using anti-occludin FITC-conjugated antibodies) is used to verify whether various lactoferrin forms affect the integrity of the intestinal epithelium barrier. Again, no difference are found for MnLf in comparison with other lactoferrin preparations (apo-Lf, native Lf, holo-Lf).

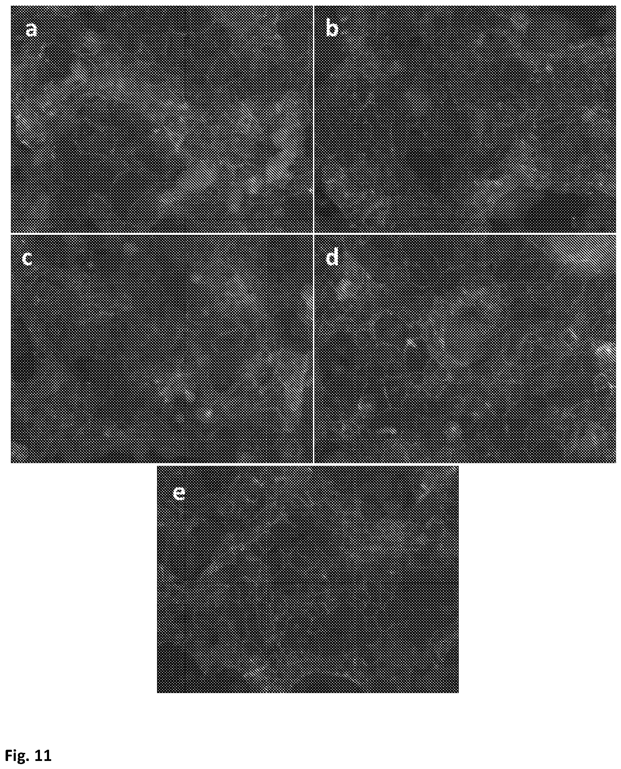

Obtained results (**Fig. 11**) points to lack of cytotoxicity of the MnLf complex.

The integrity of the intestinal epithelium is not affected as well by the complex being the subject-matter of the invention. In the performed tests, this form of Lf does not differ significantly from the commercially available lactoferrin preparation that is granted a GRAS status.

### Immunostimulatory properties of MnLf

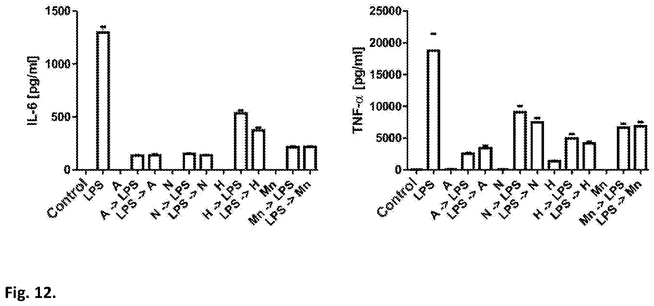

Series of experiments are performed to assay the immunostimulatory properties of the lactoferrin saturated with manganese ions. ELISA test is employed to determine the levels of pro- and anti-inflammatory cytokines release by the mammalian cells treated with various Lf forms. We observed no stimulation of cytokine secretion in the case of Caco-2 cells (cytokine levels are below detection limits of the ELISA kit used). No cytokine release is observed upon incubation with various lactoferrin forms in experiments on immune cells (using murine monocyte/macrophage cell line J774A.1, Fig. 12).

Some experiments on J774A.1 macrophages also assayed the impact of lactoferrin on pro-inflammatory cytokine secretion induced in these cells by presence of lipopolysaccharide. Macrophages are incubated with:
- LPS isolated from *Escherichia coli* (Sigma-Aldrich), 10 ng/ml
- Various Lf forms (apo-Lf, native Lf, holo-Lf, MnLf) at a concentration of 5 mg/ml

For part of the samples, the bacterial component (LPS) or a tested lactoferrin form is added to the cells. To test the interaction of various lactoferrin forms with LPS, sequential addition of LPS and Lf is studied as well. For example, LPS is added to the cells after 30 minutes of incubation with apo-Lf. Reversed sequence of additions is tested as well. The supernatants are collected after 23 hours of incubation.

Obtained results confirmed lactoferrin ability to mitigate LPS-induced secretion of pro-inflammatory cytokines by the macrophages (Fig. 12). Addition of lactoferrin before or after incubation with LPS resulted in the significant decrease in the levels of both IL-6 and TNF-α secreted by the cells (15-20% compared to the LPS alone sample). MnLf does not differ in this aspect from the commercially available and described in the literature forms of lactoferrin.

## Claims

1. A complex of lactoferrin with manganese ions, wherein manganese (III) ions are 96-98% (molar ratio) of all metal ions of the complex.

2. The complex according to claim 1, wherein the manganese (III) ions are 98% (molar ratio) of all metal ions of the complex.

3. The complex according to claims 1 or 2, wherein the lactoferrin saturation with Mn(III) is 50% or below.

4. A method of obtaining the complex according to claim 1, including a reaction of lactoferrin with manganese salt and purifying MnLf complex, wherein the reaction is carried via dialysis.

5. The method according to claim 4, wherein:
- reaction of lactoferrin with manganese salt is conducted via dialysis in a buffer chosen from: 20-100 mM HEPES with addition of 100 mM NaCl, 25 mM NaHCO₃, with pH within a range 7.0 - 7.5, at temperature within a range of 30 - 40°C, and the manganese salt is manganese(II) citrate, with molar ratio of 5 - 20 between manganese and lactoferrin, time of reaction within 12 - 48 hours,
- purifying of the complex is conducted via dialysis to ultrapure water for 10-24 hours at room temperature with 3 changes of water.

6. The method according to claim 5, wherein the molar ratio between Mn²⁺ and citric acid at pH 7.4 is set to 3:2.

7. The method according to any of the previous claims 4 - 6 wherein the reaction of lactoferrin with manganese salt is performed in aerobic conditions.

8. The method according to claim 5, wherein the buffer used is HEPES at a concentration of 50 mM.

9. The method according to claim 5, wherein the reaction of lactoferrin with manganese salt is conducted at temperature of 37°C.

10. The method according to claim 5, wherein the reaction of lactoferrin with manganese salt is conducted with at least 5-fold excess of Mn²⁺ in proportion to lactoferrin.

11. The complex according to claim 1 for use in the treatment and/or prophylaxis of gastrointestinal tract microbiota disorders.

12. The complex for use according to claim 11, wherein the disorder of the gastrointestinal tract is dysbacteriosis associated with antibiotic therapy, oncotherapy, preterm birth, inflammatory bowel diseases.

13. The complex for use according to claim 11, wherein it is to be administered to preterm infants, to patients undergoing an antibiotic therapy, to oncological patients, or to patients suffering from chronic inflammatory bowel diseases.

14. The complex according to claim 1 for use in the treatment of disorders caused by bacteria, fungi and/or viruses.

15. The complex according to claim 1 for use in the treatment of sepsis.

16. A pharmaceutical composition comprising the complex according to claims 1 to 3.

17. A dietary supplement comprising the complex according to claims 1 to 3.

18. A non-therapeutic use of the dietary supplement according to claim 17 for regulating the microbiota of the gastrointestinal tract, mainly for tipping the balance towards the probiotic bacteria.

19. The use of the complex according to claim 1 to prepare a dietary supplement.

20. The use of the complex according to claim 1 to prepare a pharmaceutical composition.

## Patentansprüche

1. Komplex von Lactoferrin und Mangan, wobei Mangan(III)-Ionen 96-98% (Molverhältnis) aller Metallionen des Komplexes ausmachen.

2. Komplex gemäß Anspruch 1, wobei die Mangan(III)-Ionen 98% (Molverhältnis) aller Metallionen des Komplexes ausmachen.

3. Komplex gemäß einem der Ansprüche 1 oder 2, wobei die Laktoferrinsättigung mit Mn(III) 50% oder weniger beträgt.

4. Verfahren zur Herstellung eines Komplexes gemäß Anspruch 1, enthaltend die Reaktion von Lactoferrin und Mangansalz und Aufreinigung des MnLf-Komplexes, wobei die Reaktion über Dialyse erfolgt.

5. Verfahren gemäß Anspruch 4, wobei:
- die Reaktion von Lactoferrin und Mangansalz über Dialyse in einem Puffer erfolgt, ausgewählt aus: 20-100 mM HEPES mit Zugabe von 100 mM NaCl, 25 mM NaHCO₃, mit einem pH-Wert im Bereich von 7,0-7,5, bei einer Temperatur im Bereich von 30-40°C, und wobei das Mangansalz Mangan(II)-citrat ist, mit einem Molverhältnis von 5-20 zwischen Mangan und Lactoferrin, Reaktionszeit von 12-48 Stunden,
- die Aufreinigung des Komplexes über Dialyse in ultrareines Wasser innerhalb von 10-24 Stunden bei Raumtemperatur, mit 3-fachem Wasserwechsel erfolgt.

6. Verfahren gemäß Anspruch 5, wobei Mn²⁺ und Zitronensäure bei pH 7,4 im Molverhältnis 3:2 eingesetzt werden.

7. Verfahren gemäß einem der vorherigen Ansprüche 4-6, wobei die Reaktion von Lactoferrin mit Mangansalz unter aeroben Bedingungen erfolgt.

8. Verfahren gemäß Anspruch 5, wobei als Puffer HEPES in einer Konzentration von 50 mM verwendet wird.

9. Verfahren gemäß Anspruch 5, wobei die Reaktion von Lactoferrin mit Mangansalz bei einer Temperatur von 37°C erfolgt.

10. Verfahren gemäß Anspruch 5, wobei die Reaktion von Lactoferrin mit Mangansalz mit einem mindestens 5-fachen Überschuss von Mn²⁺ im Verhältnis zu Lactoferrin erfolgt.

11. Komplex gemäß Anspruch 1 zur Verwendung bei der Behandlung und/oder Vorbeugung von Mikrobiota-Erkrankungen des Gastrointestinaltraktes.

12. Komplex zur Verwendung gemäß Anspruch 11, wobei die Erkrankung des Gastrointestinaltraktes eine Dysbakteriose ist, die mit Antibiotikatherapie, Onkotherapie, Frühgeburt, chronisch-entzündlichen Darmerkrankungen assoziiert ist.

13. Komplex zur Verwendung gemäß Anspruch 11, wobei dieser Frühgeborenen, Patienten, die sich einer Antibiotikatherapie unterziehen, onkologischen Patienten oder Patienten, die an chronisch-entzündlichen Darmerkrankungen leiden, zu verabreichen ist.

14. Komplex gemäß Anspruch 1 zur Verwendung bei der Behandlung von Erkrankungen, die durch Bakterien, Pilze und/oder Viren verursacht werden.

15. Komplex gemäß Anspruch 1 zur Verwendung bei der Behandlung von Sepsis.

16. Pharmazeutische Zusammensetzung enthaltend einen Komplex gemäß Ansprüchen 1 bis 3.

17. Nahrungsergänzungsmittel enthaltend einen Komplex gemäß Ansprüchen 1 bis 3.

18. Nicht-therapeutische Anwendung eines Nahrungsergänzungsmittels gemäß Anspruch 17 zur Regulierung der Mikrobiota des Gastrointestinaltraktes, hauptsächlich zur Verschiebung des Gleichgewichts in Richtung probiotischer Bakterien.

19. Verwendung eines Komplexes nach Anspruch 1 zur Herstellung eines Nahrungsergänzungsmittels.

20. Verwendung eines Komplexes nach Anspruch 1 zur Herstellung einer pharmazeutischen Zusammensetzung.

## Revendications

1. Complexe de lactoferrine avec des ions manganèse, dans lequel les ions manganèse (III) représentent 96 à 98 % (rapport molaire) de tous les ions métalliques du complexe.

2. Complexe selon la revendication 1, dans lequel les ions manganèse (III) représentent 98% (rapport molaire) de tous les ions métalliques du complexe.

3. Complexe selon l'une des revendications 1 ou 2, dans lequel la saturation de la lactoferrine avec Mn(III) est de 50 % ou moins.

4. Procédé d'obtention du complexe selon la revendication 1, comprenant une réaction de la lactoferrine avec du sel de manganèse et la purification du complexe MnLf, dans lequel la réaction est effectuée par dialyse.

5. Procédé selon la revendication 4, dans lequel:
- la réaction de la lactoferrine avec le sel de manganèse est conduite par dialyse dans un tampon choisi parmi : HEPES à 20 à 100 mM avec addition de NaCl à 100 mM, NaHCO3 à 25 mM, avec un pH se situant dans la plage allant de 7,0 à 7,5, à une température se situant dans la plage allant de 30 à 40°C, et le sel de manganèse est le citrate de manganèse (II), avec un rapport molaire de 5 à 20 entre le manganèse et la lactoferrine, temps de réaction entre 12 et 48 heures,
- la purification du complexe est conduite par dialyse avec de l'eau ultrapure pendant 10 à 24 heures à température ambiante avec 3 changements d'eau.

6. Procédé selon la revendication 5, dans lequel le rapport molaire entre Mn2+ et l'acide citrique à pH 7,4 est établi à 3 : 2.

7. Procédé selon l'une quelconque des revendications précédentes 4 à 6, dans lequel la réaction de la lactoferrine avec le sel de manganèse est effectuée dans des conditions aérobies.

8. Procédé selon la revendication 5, dans lequel le tampon utilisé est l'HEPES à une concentration de 50 mM.

9. Procédé selon la revendication 5, dans lequel la réaction de la lactoferrine avec le sel de manganèse est conduite à une température de 37°C.

10. Procédé selon la revendication 5, dans lequel la réaction de la lactoferrine avec le sel de manganèse est conduite avec un excès de Mn2+ d'au moins 5 fois par rapport à la lactoferrine.

11. Complexe selon la revendication 1 pour une utilisation dans le traitement et/ou la prophylaxie de troubles du microbiote du tractus gastro-intestinal.

12. Complexe pour l'utilisation selon la revendication 11, dans lequel le trouble du tractus gastro-intestinal est une dysbactériose associée à une antibiothérapie, une oncothérapie, une naissance prématurée, des maladies inflammatoires de l'intestin.

13. Complexe pour l'utilisation selon la revendication 11, dans lequel il doit être administré à des nourrissons prématurés, à des patients soumis à une antibiothérapie, à des patients oncologiques ou à des patients souffrant de maladies inflammatoires chroniques de l'intestin.

14. Complexe selon la revendication 1 pour une utilisation dans le traitement de troubles provoqués par des bactéries, des champignons et/ou des virus.

15. Complexe selon la revendication 1 pour une utilisation dans le traitement de la sepsie.

16. Composition pharmaceutique comprenant le complexe selon l'une des revendications 1 à 3.

17. Complément alimentaire comprenant le complexe selon l'une des revendications 1 à 3.

18. Utilisation non thérapeutique du complément alimentaire selon la revendication 17 pour réguler le microbiote du tractus gastro-intestinal, principalement pour faire pencher la balance du côté des bactéries probiotiques.

19. Utilisation du complexe selon la revendication 1 pour préparer un complément alimentaire.

20. Utilisation du complexe selon la revendication 1 pour préparer une composition pharmaceutique.
